# EUROPEAN PATENT SPECIFICATION

(11) **EP 4 302 085 B1**
(45) Date of publication and mention of the grant of the patent: **24.06.2026**
(21) Application number: 22763671.9
(22) Date of filing: 02.03.2022
(51) Int. Cl.: G01N 33/36, G01N 1/40, G01N 30/72, B01D 59/44, B01D 59/50, B01D 59/26

(54) **A METHOD FOR DETERMINING THE ORIGIN OF A MATERIAL BY CHEMICAL SEPARATION OF SYNTHETIC FIBRES FROM A BLEND OF SYNTHETIC FIBRES AND SEMI-SYNTHETIC OR NATURAL FIBRES**
VERFAHREN ZUR BESTIMMUNG DER HERKUNFT EINES MATERIALS DURCH CHEMISCHE TRENNUNG VON SYNTHETISCHEN FASERN AUS EINER MISCHUNG VON SYNTHETISCHEN FASERN UND HALBSYNTHETISCHEN ODER NATÜRLICHEN FASERN
PROCÉDÉ POUR DÉTERMINER L'ORIGINE D'UN MATÉRIAU PAR SÉPARATION CHIMIQUE DE FIBRES SYNTHÉTIQUES D'UN MÉLANGE DE FIBRES SYNTHÉTIQUES ET DE FIBRES SEMI-SYNTHÉTIQUES OU NATURELLES

(30) Priority: 03.03.2021 NZ 21773526
(43) Date of publication of application: 10.01.2024
(73) Proprietor: Oritain Global Limited, Dunedin, 9016 (NZ)
(72) Inventor: FREW, Russell David, Dunedin, 9081 (NZ)
(74) Representative: Forresters IP LLP
(86) International application number: PCT/NZ2022/050023
(87) International publication number: WO 2022/186705

(56) References cited:
- US-A1- 2015 377 854
- SUZUKI YAEKO ET AL: "A Novel Method to Discriminate between Natural and Synthetic Fibers by Stable Carbon, Nitrogen, and Oxygen Isotope Analyses", CHEMISTRY LETTERS, vol. 41, no. 3, 25 February 2012 (2012-02-25), pages 242 - 243, XP093230936, ISSN: 0366-7022, DOI: 10.1246/cl.2012.242
- MEIER-AUGENSTEIN WOLFRAM ET AL: "Discrimination of unprocessed cotton on the basis of geographic origin using multi-element stable isotope signatures : Discrimination of unprocessed cotton", RAPID COMMUNICATIONS IN MASS SPECTROMETRY, vol. 28, no. 5, 29 January 2014 (2014-01-29), GB, pages 545 - 552, XP055968060, ISSN: 0951-4198, DOI: 10.1002/rcm.6811
- BEUSSMAN DOUGLAS J.: "The Analysis of Trace Forensic Evidence Using Isotope Ratio Mass Spectrometry: Differentiating Fibers", OFFICE OF JUSTICE PROGRAMS, 1 October 2017 (2017-10-01), XP055967838, [retrieved on 20221004]
- SUNGUR ŞANA, GÜLMEZ FATIH: "Determination of Metal Contents of Various Fibers Used in Textile Industry by MP-AES", JOURNAL OF SPECTROSCOPY, vol. 2015, 1 January 2015 (2015-01-01), pages 1 - 5, XP055967843, ISSN: 2314-4920, DOI: 10.1155/2015/640271
- MEIER-AUGENSTEIN WOLFRAM, KEMP HELEN F., SCHENK EMILY R., ALMIRALL JOSE R.: "Discrimination of unprocessed cotton on the basis of geographic origin using multi-element stable isotope signatures : Discrimination of unprocessed cotton", RAPID COMMUNICATIONS IN MASS SPECTROMETRY, JOHN WILEY & SONS, GB, vol. 28, no. 5, 29 January 2014 (2014-01-29), GB , pages 545 - 552, XP055968060, ISSN: 0951-4198, DOI: 10.1002/rcm.6811
- MEIER-AUGENSTEIN W, DAEID N N, KEMP H F: "INVESTIGATING THE ORIGIN OF COTTON IN YARN USING MULTIVARIATE ISOTOPE PROFILES", PROCEEDINGS OF THE 31 ST INTERNATIONAL COTTON CONFERENCE, 1 January 2012 (2012-01-01), XP055968033, Retrieved from the Internet <URL:https://baumwollboerse.de/wp-content/uploads/vortraege/Vortrag-Augenstein_2012.pdf> [retrieved on 20221005]
- SCHENK EMILY R.: "Geographic Provenancing of Unprocessed Cotton Using Elemental Analysis and Stable Isotope Ratios", DISSERTATION, FLORIDA INTERNATIONAL UNIVERSITY, 1 January 2012 (2012-01-01), FLORIDA INTERNATIONAL UNIVERSITY, XP055968034, Retrieved from the Internet <URL:/https://digitalcommons.fiu.edu/cgi/viewcontent.cgi?referer=&httpsredir=1&article=1867&context=etd> [retrieved on 20221005]
- GENTILE NATACHA; SIEGWOLF ROLF T.W.; ESSEIVA PIERRE; DOYLE SEAN; ZOLLINGER KURT; DELÉMONT OLIVIER : "Isotope ratio mass spectrometry as a tool for source inference in forensic science: A critical review", FORENSIC SCIENCE INTERNATIONAL, ELSEVIER B.V., AMSTERDAM, NL, vol. 251, 9 April 2015 (2015-04-09), AMSTERDAM, NL , pages 139 - 158, XP029157371, ISSN: 0379-0738, DOI: 10.1016/j.forsciint.2015.03.031
- "Forensic Examination of Fibres. 3rd. Ed.", 20 December 2017, CRC PRESS, US, ISBN: 978-1-315-15658-3, article JAMES ROBERTSON, CLAUDE ROUX, KENNETH G. WIGGINS: "Chapter 9.3. Isotope Ratio Mass Spectrometry (IRMS)", pages: 317 - 319, XP009550210, DOI: 10.1201/9781315156583
- ZIEGLER STEFAN, COSTA FELIPE, SCHMIESTER JOHANNES, NEWTON JASON,: "Tracing the origin of cotton: a novel approach to increase transparency of cotton supply chains", INTERNATIONAL COTTON CONFERENCE BREMEN, 17 March 2021 (2021-03-17) - 19 March 2021 (2021-03-19), XP055968042
- LEHR AMY K.: "New Approaches to Supply Chain Traceability: Implications for Xinjiang and Beyond", REPORT OF THE CENTER FOR STRATEGIC AND INTERNATIONAL STUDIES (CSIS), 1 November 2020 (2020-11-01), XP055968050, Retrieved from the Internet <URL:https://www.jstor.org/stable/pdf/resrep26936.1.pdf> [retrieved on 20221005]

## Description

### TECHNICAL FIELD

The invention relates generally to a method for separating synthetic fibres from a blend of synthetic fibres with semi-synthetic or natural fibres. In particular, the invention provides a chemical method of removing synthetic fibre material from a blend while maintaining the integrity of the semi-synthetic or natural fibre material for analysis to determine the origin of the semi-synthetic or natural fibre.

### BACKGROUND OF THE INVENTION

Determining the origin of products is becoming increasingly important worldwide. Consumer awareness of the origin of foods, in particular, is significant for many markets. There is a growing need for manufacturers and suppliers to know the origin of raw materials and ingredients and to able to prove origin through scientifically reliable traceability techniques.

Verification of the origin of fibres used in the clothing and textile industries is also becoming increasingly important. Sustainability and the ethical production of certain types of fibre impact heavily on the reputation of products, brands, manufactures and suppliers. There is therefore a need to verify the origin of fibre products and raw materials using forensic science.

Cotton is one natural fibre that can be analysed chemically to determine its origin. Forensic chemistry and statistics can be combined to establish an inherent "fingerprint" based on naturally occurring chemical elements and their isotopes in the cotton and, in some cases, also on soil composition and other environmental factors. The cotton is typically cleaned and then a sample is either digested in acid for trace metal analysis or combusted/pyrolised to the relevant gas for stable isotope measurements. The tested sample data are then compared to a database of similar measurements made on origin-authenticated samples. The process works well for raw or single-origin samples.

However, difficulties arise when there is a need to establish or verify the origin of a material manufactured from cotton (or some other natural or semi-synthetic fibre) and a synthetic fibre such as polyester, a polyether-polyurea copolymer such as elastane, or nylon. The presence of the synthetic fibre interferes with establishing the fingerprint of the cotton component. Techniques for the chemical separation of synthetic and natural fibres in a material are known. However, the chemical processes used are generally for the purpose of recycling the synthetic fibre component and lead to alteration of the natural fibre such that the integrity of the natural fibre for any traceability determination is lost. The difficulties exist not only for natural fibre blends with synthetic fibres, but also with blends of natural fibres and semi-synthetic fibres such as viscose, modal, rayon, acetate, lyocell and cupro.

Suzuki Yaeko et al, a scientific paper, discloses a method to discriminate between natural and synthetic fibres by stable carbon, nitrogen and oxygen isotope analysis. (CHEMISTRY LETTERS, vol. 41, no.3, 25 February 2012, pages 242 - 243).

There is, therefore, a need for safe and reliable chemical processing methods that enable the separation of natural fibres or semi-synthetic fibres from synthetic fibres so that the natural or semi-synthetic fibres can be reliably analysed to determine origin.

### SUMMARY OF THE INVENTION

The invention described and claimed herein have many attributes and examples including, but not limited to, those set forth or described or referenced in this Summary of the Invention. It is not intended to be all-inclusive and the invention described and claimed herein is not limited to or by the features or examples identified in this Summary of the Invention, which is included for purposes of illustration only and not restriction.

In one aspect of the invention, there is provided a method for determining the origin of a material comprising a blend of synthetic fibres and natural or semi-synthetic fibres comprising the steps:
a. contacting a sample of the material with an extractant solution to form a solution or suspension comprising i) synthetic fibres and fragments of the synthetic fibres, and ii) a solid residue containing natural or semi-synthetic fibres;
b. separating the solution formed in step a. from the solid residue;
c. drying the solid residue;
d. determining isotope ratios of one or more of the elements carbon, oxygen, hydrogen, nitrogen, sulfur and strontium in the solid residue, and/or determining the concentrations of one or more trace elements in the solid residue; and
e. comparing the isotope ratios and/or concentrations determined in step d. against data for natural or semi-synthetic fibres of known origin to determine the origin of the material.

In some embodiments of the invention, the natural fibres are fibres of cotton, wool, fur, silk, hemp, linen or jute. The wool may originate from any wool-producing animal, including sheep, goats (e.g. cashmere, angora, mohair), alpaca, and llamas. The fur may originate from any fur-producing animal, including rabbits and possums.

In some embodiments of the invention, the semi-synthetic fibres are fibres of viscose, modal, rayon, acetate, lyocell or cupro.

In some embodiments of the invention, the synthetic fibres are fibres of polyester, nylon, elastane or acrylic.

In some embodiments of the invention, the sample of material has been milled to an average particle size of about 50-250 microns before contacting with the extractant solution in step a.

In some embodiments of the invention, the extractant solution is ethanol, dimethylformamide (DMF), phenol, dichloromethane (DCM), aqueous hydrochloric acid, aqueous sodium hydroxide, or formic acid, or any combination thereof.

In some embodiments of the invention step a. comprises one or more cycles of i) agitating the sample in the extractant solution, ii) centrifuging the sample in the extractant solution, and iii) separating the solution from the solid residue.

In some embodiments of the invention, the method further comprises one or more steps of heating the sample at a temperature in the range 50-90 °C, for example, 70 °C.

In some embodiments of the invention, the solution formed in step a. is separated from the solid residue by decanting, filtering or aspirating.

In some embodiments of the invention, the synthetic fibre is polyester, and the extractant solution is phenol in DCM (20% w/v) or aqueous sodium hydroxide (10% w/v).

In some embodiments of the invention, the synthetic fibre is elastane, and the extractant solution is ethanol in DMF (10% v/v).

In some embodiments of the invention, the synthetic fibre is nylon, and the extractant solution is aqueous hydrochloric acid (18% w/v) or formic acid (98% v/v).

In some embodiments of the invention, the synthetic fibre is polyester and the extractant solution is aqueous sodium hydroxide (10 % w/v).

In some embodiments of the invention, the material is a fabric or a textile.

In some embodiments of the invention, the origin is the country or region of a country where the natural or semi-synthetic fibre was grown or produced.

### BRIEF DESCRIPTION OF THE FIGURE

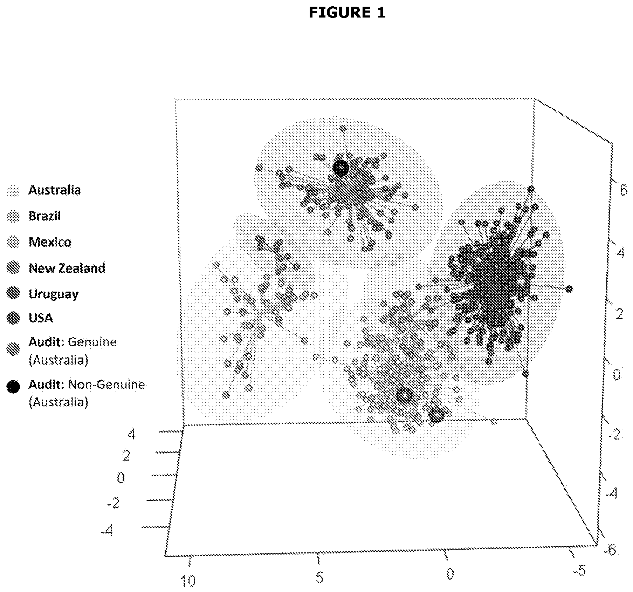

Figure 1 is a representation of trace metal and stable isotope data in multivariate space.

### DEFINITIONS

For the purposes of the invention disclosed herein, the following terms shall have the following meanings.

The term "origin" means the geographical location where a fibre was grown or produced.

The term "natural fibre" means fibre produced by plants or animals and includes, but is not limited to, cotton, silk, hemp, linen, jute, wool and fur.

The term "semi-synthetic fibre" means fibre produced by plants or animals that has been chemically modified or partially degraded and includes, but is not limited to, viscose, modal, rayon, acetate, lyocell and cupro.

The term "synthetic fibre" means a polymer chemically synthesised from small molecules and spun into a fibre and includes, but it not limited to, polyester, nylon, elastane, and acrylic.

The term "extractant solution" means a liquid used to chemically separate a constituent of a mixture by dissolution, degradation or suspension.

The term "isotope ratio" means the ratio of atomic abundances of two or more isotopes of the same chemical element.

The term "trace element" means a chemical element whose concentration is very low and includes, but is not limited to, an element having a concentration of less than 100 ppm or less than 100 µg/g. The term "trace metal" has a correspnding meaning.

Unless specifically defined otherwise, all technical and scientific terms used herein shall be taken to have the same meaning as commonly understood by one of ordinary skill in the art.

It is intended that reference to a range of numbers disclosed herein (e.g. 1 to 10) also incorporates reference to all related numbers within that range (e.g. 1, 1.1, 2, 3, 3.9, 4, 5, 6, 6.5, 7, 8, 9 and 10) and also any range of rational numbers within that range (for example 2 to 8, 1.5 to 5.5 and 3.1 to 4.7) and, therefore, all sub-ranges of all ranges expressly disclosed herein are expressly disclosed. These are only examples of what is specifically intended and all possible combinations of numerical values between the lowest value and the highest value enumerated are to be considered to be expressly stated in this application in a similar manner.

The term "and/or", e.g., "X and/or Y", shall be understood to mean either "X and Y" or "X or Y" and shall be taken to provide explicit support for both meanings or for either meaning.

The term "a" or "an" may refer to one or more than one of the entity specified. As such, the terms "a" or "an", "one or more" and "at least one" can be used interchangeably.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

### DETAILED DESCRIPTION

The invention relates to a method for determining the origin of a material comprising a blend of synthetic fibres and natural or semi-synthetic fibres. Synthetic fibres can be separated from a blend with natural or semi-synthetic fibres, but the difficulty with previously known techniques has been achieving this while preserving the integrity of the natural or semi-synthetic fibres for the purpose of chemical analysis and traceability or origin and authenticity investigations. Most known forms of chemical treatment of fibre blends alter the ratios of stable isotopes of chemical elements in natural and semi-synthetic fibres, which means that isotope values cannot be used to reliably determine the origin of the fibre. The applicants have found a way to achieve this separation and reliably determine the origin of the material.

The method of the invention comprises the following steps:
a. contacting a sample of the material with an extractant solution to form a solution or suspension comprising i) synthetic fibres and fragments of the synthetic fibres, and ii) a solid residue containing natural or semi-synthetic fibres;
b. separating the solution or suspension formed in step a. from the solid residue;
c. drying the solid residue;
d. determining isotope ratios of one or more of the elements carbon, oxygen, hydrogen, nitrogen, sulfur and strontium in the solid residue, and/or determining the concentrations of one or more trace elements in the solid residue; and
e. comparing the isotope ratios and/or concentrations determined in step d. against data for natural or semi-synthetic fibres of known origin to determine the origin of the material.

The extractant solution and the conditions under which the sample is exposed to the extractant solution (e.g. temperature and time) are selected on the basis of the type of synthetic fibre that must be removed from the blend. For example, in one embodiment of the invention, the applicant has found that a solution of phenol in dichloromethane (DCM) is effective when the synthetic fibre is polyester. In another embodiment, the applicant found that a solution of ethanol in dimethyl formamide (DMF) is effective when the synthetic fibre is elastane. In a further embodiment, the applicant found that a solution of aqueous hydrochloric acid is effective when the synthetic fibre is nylon. In each embodiment, the isotope values of key elements were preserved as verified by independent experiments.

It will be appreciated that any of a wide range of organic or inorganic liquids or solvents, or combinations of liquids or solvents, may be used depending on the synthetic fibre to be removed and the natural or semi-synthetic fibre to be preserved for traceability analysis.

Contact times and temperatures are also important for some embodiments of the invention. For example, the sample and extractant solution may be stirred at a temperature of 50-90 °C, e.g. 70 °C, for 1-2 hours or longer.

During step a., the majority, if not all, the synthetic fibres dissolve into the extractant solution or become suspended in the extractant solution or at least some of the synthetic fibres are broken down into smaller fragments which dissolve into the extractant solution or become suspended in the extractant solution.

The solution or suspension formed in step a. is then separated from any remaining solid material. One way to do this is to centrifuge and then decant the solution leaving a solid residue which is then dried before chemical analysis. Any other separation technique, such as filtration, may be used.

The separation process may also include one or more stages of washing with a volatile solvent such as ethanol to remove all traces of extractant solution components such as phenol or DMF.

Once the solid residue has been dried, it is ready for chemical analysis to determine isotope ratios of one or more chemical elements in the solid residue and/or determine the concentrations of one or more chemical elements in the solid residue.

Stable isotope ratios can be determined by conversion of the sample to a gas and then measurement of the ratio of masses in an isotope ratio mass spectrometer (IRMS) or by cavity ring-down spectroscopy (CRDS). Samples are typically dried and then decomposed at high temperatures under an inert atmosphere, such as helium, to produce hydrogen and carbon monoxide gases. These are subjected to chromatography and the ratios of isotope masses measured. Combustion of the sample in oxygen at high temperatures produces carbon, nitrogen and sulfur gases. These gases are separated by gas chromatography and swept into the source of the IRMS, where the relative abundances of their isotope masses are measured.

Strontium isotope ratios can be determined using a multi-collector inductively coupled mass spectrometer after the sample has been subjected to column chromatography to remove interferences caused by the presence of other elements such as rubidium.

The concentrations of trace metals, such as Mg, Al, K, Ca, V, Cr, Mn, Fe, Co, Ni, Cu, Zn, Se, As, Rb, Sr, Cd, Cs, Ba, Pb and U, can be determined by decomposition of the sample in concentrated mineral acids with heating. Once a clear solution is achieved, the sample is dried on a hot plate, and the residue redissolved in dilute HNO₃ (2 M). A known amount of an internal standard (e.g. Rh) is added, and the trace metals in the diluted solution are quantified using quadrupole inductively coupled mass spectrometry.

Verification of the origin of a sample is achieved by connecting the stable isotope ratios and/or the trace element concentration data with database information for origin-authenticated fibres. As numerous parameters are measured both univariate and multivariate statistics are used to interpret the data. Univariate exploration (e.g. box and whisker graphs, x-y plots) is used to identify groupings and outliers and test the assumptions the multivariate models require of the data (e.g. normal distribution). Transformation (e.g. log) procedures are applied as required. Several different models (usually 8) are applied for interpretation. These include Principal Component Analysis for data exploration, several discriminate models (Linear Discriminate Analysis, Quadratic Discriminate Analysis, Random Forest, Artificial Neural Networks) and others.

The invention is further described with reference to the Examples. It will be appreciated that the invention as claimed is not intended to be limited in any way by the Examples.

Examples 1 and 2 describe methods for determining stable isotope ratios of chemical elements in a sample and the concentrations of trace metals.

Example 3 describes how polyester fibre can be extracted from a blend with cotton using an extractant solution of 20% phenol in DCM (w/v). It will be appreciated that the same or similar procedure can be used to extract polyester fibre from a blend with other types of natural fibres such as wool fibres. Example 3 shows that the treatment of the cotton/polyester blend was effective in removing the polyester such that the after-treatment stable isotope values for C, O and H were indistinguishable from the original cotton, i.e. they were within the analytical uncertainty of the measurement. This means that the stable isotope measurements for the treated cotton do reliably represent the original isotope values of the cotton and can therefore be used in determining its origin.

Example 4 describes how nylon can be extracted from a blend with cotton. The results presented in Table 2 show that the treatment of cotton with aqueous HCl (18% w/v) under the described conditions does not affect the stable isotope values of C, O or H of the cotton. The presence of nylon in the blend with cotton does affect the stable values rendering them unusable for determining origin. Example 4 shows that the treatment of the cotton/nylon blend was effective in removing the nylon such that the after-treatment stable isotope values for C, O and H were indistinguishable from the original cotton, i.e. they were within the analytical uncertainty of the measurement. This means that the stable isotope measurements for the treated cotton do reliably represent the original isotope values of the cotton and can therefore be used in determining its origin.

The outcome is the same for the extraction of elastane from a blend with cotton, as shown in Example 5. The results presented in Table 3 show that the treatment of cotton with DMF under the described conditions does not affect the stable isotope values of C, O or H of the cotton. The presence of elastane in the blend with cotton does affect the stable values rendering them unusable for determining origin. Example 5 therefore shows that the treatment of the cotton/elastane blend was effective in removing the elastane such that the after-treatment stable isotope values for C, O and H were indistinguishable from the original cotton, i.e. they were within the analytical uncertainty of the measurement. This means that the stable isotope measurements for the treated cotton do reliably represent the original isotope values of the cotton and can therefore be used in determining its origin.

Example 6 describes the extraction of two synthetic fibres from a blend with cotton. The results presented in Table 4 show that both elastane and polyester can be removed while leaving the cotton stable isotope values unaffected. The treatment of the cotton/polyester/elastane blend was effective in removing the polyester and elastane such that the after-treatment stable isotope values for C, O and H were indistinguishable from the original cotton i.e. they were within the analytical uncertainty of the measurement. Again, the stable isotope measurements for the cotton reliably represent the original isotope values of the cotton and can be used in determining its origin.

Example 7 also describes the extraction of two synthetic fibres from a blend with cotton. The results of Table 5 show that a blend of cotton with elastane and nylon can be treated to remove both types of synthetic fibre and leave the cotton stable isotope values unaffected. The stable isotope measurements for the treated cotton represent the original isotope values of the cotton and can be used in determining its origin.

Example 8 describes the extraction of polyester from a blend with wool. In this method the wool remains in the suspension with dichoromethane whereas the polyester is denser and separated by centrifugation. Repeated decanting following centrifugation effectively removes the polyester from the wool. The results of Tables 6 and 7 show that a blend of wool and polyester can be treated to remove the polyester and leave the wool stable isotope values unaffected. The stable isotope measurements of the treated wool represent the original isotope values of the wool and can be used in determining its origin.

Example 9 describes how nylon can be extracted from a blend with wool. The results presented in Tables 8 and 9 show that the treatment of wool with Formic acid (98%) under the described conditions does not affect the stable isotope values of N, C, S, O or H of the wool. Example 9 shows that the treatment of the wool/nylon blend was effective in removing the nylon such that the after-treatment stable isotope values for N, C, S, O and H were indistinguishable from the original cotton, i.e. they were within the analytical uncertainty of the measurement. This means that the stable isotope measurements for the treated wool do reliably represent the original isotope values of the wool and can therefore be used in determining its origin
Example 10 describes how elastane can be extracted from a blend with wool. The outcome is the same for the extraction of elastane from a blend with cotton, as shown in Example 5. The results presented in Tables 10 and 11 show that the treatment of wool with DMF under the described conditions does not affect the stable isotope values of N, C, S, O or H of the wool. The presence of elastane in the blend with wool does affect the stable values rendering them unusable for determining origin. Example 10 therefore shows that the treatment of the wool/elastane blend was effective in removing the elastane such that the after-treatment stable isotope values for N, C, S, O and H were indistinguishable from the original wool, i.e. they were within the analytical uncertainty of the measurement. This means that the stable isotope measurements for the treated wool do reliably represent the original isotope values of the wool and can therefore be used in determining its origin.

Example 11 also describes how acrylic can be extracted from a blend with cotton. The presence of acrylic in the blend with cotton does affect the stable values rendering them unusable for determining origin. Example 11 therefore shows that the treatment of the cotton/acrylic blend with DMF was effective in removing the acrylic such that the after-treatment stable isotope values for C, O and H were indistinguishable from the original cotton, i.e. they were within the analytical uncertainty of the measurement. This means that the stable isotope measurements for the treated cotton do reliably represent the original isotope values of the cotton and can therefore be used in determining its origin.

Example 12 describes how polyester fibre can be extracted from a blend with cotton using an extractant solution of sodium hydroxide (10% w/v). Example 12 shows that the treatment of the cotton/polyester blend was effective in removing the polyester such that the after-treatment stable isotope values for C, O and H were indistinguishable from the original cotton, i.e. they were within the analytical uncertainty of the measurement. This means that the stable isotope measurements for the treated cotton do reliably represent the original isotope values of the cotton and can therefore be used in determining its origin

Example 13 shows how the data obtained from a sample can be compared with data from authentic samples to determine the origin of the sample tested.

### EXAMPLES

### Example 1: Stable isotope analysis

The determination of stable isotope ratios in a sample was achieved by conversion of the sample to a suitable gas and then the measurement of the ratio of masses in an isotope ratio mass spectrometer (IRMS). For oxygen and hydrogen isotope ratios, a small aliquot (~500 µg) was weighed into a silver capsule. The sample was dried under vacuum for four days and then decomposed by thermolysis in a helium atmosphere. The resulting H₂ and CO gases were separated in time through a chromatography column, and the ratio of relevant masses (3:2 for HD:H₂, 30:28 for the ¹⁸O:¹⁶O in CO, and 29:28 for the ¹³C:¹²C in CO) measured. The process used for C, N and S was similar, except the decomposition of the sample was achieved by combustion in oxygen at 1020 °C. Samples for strontium isotope ratio determination were digested similarly to the trace metal samples. The dried residue was redissolved in HNO₃ (8 M) and loaded onto a column of Sr-specific resin. The other elements in the sample were washed through the resin and only the Sr was retained. After washing the Sr was eluted off the resin with dilute HNO₃ (0.1 M). The eluent containing the Sr was dried and the residue redissolved in HNO₃ (2 M). The ratios of ⁸⁸Sr, ⁸⁷Sr, and ⁸⁶Sr were measured on a multi-collector inductively coupled mass spectrometer.

### Example 2: Trace metal analysis

Trace metal concentrations in a sample were determined after decomposition of the sample in concentrated mineral acids (HNO₃ and/or HCl) with heating. Once a clear solution formed the acid was dried off and the residue redissolved in 2% HNO₃. A known amount of internal standard (e.g. Rh) was added and the solution diluted to a pre-determined volume with ultra-pure water. The trace metals in the diluted solution were quantified using quadrupole inductively coupled mass spectrometry.

### Example 3: Polyester extraction from a blend with cotton using phenol/DCM

A piece of fabric (polyester blended with cotton) was homogenised by milling to samples having an approximate particle size of 50-250 µm. A sample (100 mg) was added to a 15 ml polypropylene centrifuge tube and phenol/DCM (20% v/v, 5 ml) added before tightly capping the tube. The tube was vortexed for 2 min and then placed in a water bath at 70 °C for 2 hours. The tube was then centrifuged for 5 minutes at 2000 g. After decanting the supernatant, DCM (2 ml) was added to rinse the solid residue. Following vortexing for 2 min, centrifuging for 5 min at 2000 g , and decanting, ethanol (2 ml) was added to rinse the solid residue. After repeated cycles of decanting, vortexing, centrifuging and decanting, the solid residue was oven-dried at 60 °C for 12 hours. Comparative results of stable isotope determinations and database information for authentic origin samples are shown in Table 1.

**Table 1: Comparison of stable isotope measurements for polyester/cotton blend sample and authentic origin samples**

| | δ²H | δ¹⁸O | δ¹³C |
|---|---|---|---|
| Original (100% cotton) | -42.9 | 22.8 | -26.9 |
| n=9 | 1.87 | 0.57 | 0.10 |
| After treatment | -39.93 | 22.53 | -26.7 |
| n=9 | 2.64 | 0.23 | 0.25 |
| Difference | 2.97 | -0.27 | 0.21 |
| | | | |
| Polyester/cotton (50:50) | -46.75 | 15.93 | -27.46 |
| After treatment | -43.2 | 22.5 | -27.3 |
| n=9 | 1.50 | 0.35 | 0.34 |
| Difference | -0.3 | -0.3 | -0.4 |

### Example 4: Nylon extraction from a blend with cotton using HCl

A piece of fabric (nylon blended with cotton) was homogenised by milling to samples having an approximate particle size of 50-250 µm. A sample (100 mg) was added to a 15 ml polypropylene centrifuge tube, and HCl (18% w/v, 5 ml) added before tightly capping the tube. The tube was vortexed for 2 min and immediately centrifuged for 5 min at 2000 g. After decanting the supernatant, HCl (9% w/v, 5 ml) was added to rinse the solid residue. Following vortexing for 2 min, centrifuging for 5 min at 2000 g, and decanting, ethanol (10 ml) was added to rinse the solid residue. After two further cycles of decanting, vortexing, centrifuging and decanting, the solid residue was oven-dried at 60 °C for 12 hours. Comparative results of stable isotope determinations and database information for authentic origin samples are shown in Table 2.

**Table 2: Comparison of stable isotope measurements for nylon/cotton blend sample and authentic origin samples**

| | δ²H | δ¹⁸O | δ¹³C |
|---|---|---|---|
| Original (100% cotton) | -39.3 | 23.50 | -26.76 |
| n=3 | 0.85 | 0.08 | 0.22 |
| After treatment | -42.03 | 24.41 | -26.57 |
| n=9 | 1.51 | 0.22 | 0.14 |
| Difference | -2.76 | -0.91 | -0.22 |
| | | | |
| Nylon/cotton (50:50) | -105.5 | -2.18 | -25.54 |
| After treatment | -43.49 | 23.11 | -26.81 |
| n=9 | 1.5 | 0.37 | 0.12 |
| Difference | -4.2 | -0.39 | 0.05 |

### Example 5: Elastane extraction from a blend with cotton using DMF

A piece of fabric (elastane blended with cotton) was homogenised by milling to samples having an approximate particle size of 50-250 µm. A sample (100 mg) was added to a 15 ml polypropylene centrifuge tube and 5 ml of dimethyl formamide (DMF) added before tightly capping the tube. The tube was vortexed for 2 min and then placed in a water bath at 70 °C for 2 hours. The tube was then centrifuged for 5 min at 2000 g. After decanting the supernatant, a solution of DMF:ethanol (9: 1 v/v, 5 ml) was added to rinse the solid residue. Following vortexing for 2 min, centrifuging for 5 min at 2000 g, and decanting, the rinse with DMF:ethanol was repeated. Two additional rinses with ethanol (5 ml) were conducted to ensure complete removal of the elastane and DMF from the solid residue. After repeated cycles of decanting, vortexing, centrifuging and decanting, the solid residue was oven-dried at 60 °C at least 12 hours. Comparative results of stable isotope determinations and database information for authentic origin samples are shown in Table 3.

**Table 3: Comparison of stable isotope measurements for elastane/cotton blend sample and authentic origin samples**

| | δ²H | δ¹⁸O | δ¹³C |
|---|---|---|---|
| Original (100% cotton) | -43.4 | 23.51 | -27.13 |
| n=3 | 2.44 | 0.30 | 0.02 |
| After treatment | -43.6 | 23.55 | -27.18 |
| n=9 | 2.40 | 0.25 | 0.11 |
| Difference | -0.02 | -0.04 | -0.05 |
| | | | |
| Elastane/cotton (20:80) | -118.2 | 20.93 | -26.8 |
| After treatment | -38.33 | 22.99 | -27.21 |
| n=9 | 1.8 | 0.25 | 0.12 |
| Difference | -4.2 | -0.52 | 0.09 |

### Example 6: Polyester and elastane extraction from a blend with cotton using phenol/DCM

A piece of fabric (elastane and polyester blended with cotton) was homogenised by milling to samples having an approximate particle size of 50-250 µm. Polyester was extracted according to the procedure of Example 3 above. Elastane was extracted according to the procedure of Example 5 above. The results are shown in Table 4.

**Table 4: Comparison of stable isotope measurements for polyester/elastane/cotton blend sample and authentic origin samples**

| | δ²N | %H | δ¹⁸O | %O | δ¹³C | %C |
|---|---|---|---|---|---|---|
| Original (100% cotton) n=8 | -41.2 | 5.65 | 24.89 | 18.34 | -26.73 | 36.67 |
| SD | 2.1 | 0.13 | 0.42 | 0.41 | 0.15 | 0.81 |
| After treatment | | | | | | |
| Polyester/elastane/cotton n=9 | -44.47 | 5.69 | 25.13 | 18.48 | -26.77 | 36.97 |
| SD | 1.5 | 0.12 | 0.34 | 0.34 | 0.07 | 0.68 |
| Difference | 3.3 | -0.04 | -0.24 | -0.15 | 0.04 | -0.29 |

### Example 7: Nylon and elastane extraction from a blend with cotton using HCl

A piece of fabric (nylon and elastane blended with cotton) was homogenised by milling to samples having an approximate particle size of 50-250 µm. Nylon was extracted according the procedure of Example 4 above. Elastane was extracted according the procedure of Example 5 above. The results are shown in Table 5.

**Table 5: Comparison of stable isotope measurements for nylon/elastane/cotton blend sample and authentic origin samples**

| | δ²N | %H | δ¹⁸O | %O | δ¹³C | %C |
|---|---|---|---|---|---|---|
| Original (100% cotton) n=9 | -41.30 | 6.55 | 24.93 | 21.38 | -26.91 | 42.75 |
| SD | 2.9 | 0.27 | 0.25 | 0.89 | 0.14 | 1.77 |
| After treatment | | | | | | |
| Nylon/elastane/cotton n=9 | -44.77 | 6.55 | 24.77 | 20.95 | -27.15 | 41.90 |
| SD | 2.0 | 0.28 | 0.17 | 0.97 | 0.08 | 1.95 |
| Difference | 3.5 | 0.00 | 0.16 | 0.43 | 0.24 | 0.86 |

### Example 8: Polyester extraction from a blend with wool using DCM

Three blends (95%/5% wool/polyester, 80%/20% wool/polyester, and 50%/50% wool/polyester [w/w]) were created from ground wool and polyester. The blends were homogenised for 3 min using a Retsch MM 400 (frequency: 30/s, no balls). Dichloromethane (DCM, 5 ml) was added to a 100 mg sample of each blend and raw wool (in triplicate) in a centrifuge tube and vortexed for 5 min followed by sonication (5 min). The samples were centrifuged at 4000 g for 5 min. The supernatant (wool suspended in DCM) was decanted into a 2nd centrifuge tube and vortexed for 2 min to promote separation of any residual polyester. The samples were again centrifuged at 4000 g for 5 min and the supernatant (DCM and wool) decanted into a 3rd centrifuge tube (Residue 3). The tubes were placed in a 70 °C water bath with the caps loosened until the DCM was evaporated. Residue 3 was rinsed twice with ethanol (5 ml). Fibres were dried overnight at 60 °C before IRMS analysis. The results are shown in Tables 6 and 7.

**Table 6: Comparison of stable isotope (H and O) measurements for polyester/wool blend samples**

| | d²H | | d¹⁸O | |
|---|---|---|---|---|
| | Average | StDev | Average | StDev |
| Wool untreated (n=6,5) | -80.95 | 1.17 | 13.55 | 0.21 |
| Wool treated (n=18,18) | -84.47 | 1.53 | 13.62 | 0.59 |
| Difference | -3.52 | | 0.07 | |
| | | | | |
| Polyester untreated (n=6,6) | -54.16 | 3.49 | 12.15 | 0.26 |
| 95%/5% Wool/Polyester untreated (n=6,6) | -79.99 | 1.54 | 13.38 | 0.15 |
| 95%/5% Wool/Polyester treated (n=18,16) | -84.28 | 0.91 | 13.55 | 0.65 |
| Difference from untreated wool | -3.34 | | 0.00 | |
| | | | | |
| 80%/20% Wool/Polyester untreated (n=6,6) | -75.69 | 1.50 | 12.94 | 0.22 |
| 80%/20% Wool/Polyester treated (n=18,14) | -83.82 | 1.19 | 13.36 | 0.80 |
| Difference from untreated wool | -2.88 | | -0.19 | |
| | | | | |
| 50%/50% Wool/Polyester untreated (n=3,3) | -70.12 | 1.05 | 12.35 | 0.26 |
| 50%/50% Wool/Polyester treated (n=18,17) | -84.01 | 1.03 | 13.49 | 0.77 |
| Difference from untreated wool | -3.07 | | -0.06 | |

**Table 7: Comparison of stable isotope (N, C and S) measurements for polyester/wool blend samples**

| | d¹⁵N | | d¹³C | | d³⁴S | |
|---|---|---|---|---|---|---|
| | Average | StDev | Average | StDev | Average | StDev |
| Wool untreated (n=6,6,6) | 5.97 | 0.06 | -27.54 | 0.06 | 15.73 | 0.23 |
| Wool treated (n=18,18,18) | 6.00 | 0.10 | -27.61 | 0.19 | 15.69 | 0.31 |
| Difference | 0.03 | | -0.06 | | -0.04 | |
| | | | | | | |
| Polyester untreated (n=0,3,3) | | | -28.25 | 0.08 | 15.46 | 0.67 |
| 95%/5% Wool/Polyester untreated (n=5,5,4) | 5.92 | 0.09 | -27.74 | 0.09 | 15.83 | 0.29 |
| 95%/5% Wool/Polyester treated (n=18,18,18) | 5.96 | 0.08 | -27.62 | 0.14 | 15.64 | 0.36 |
| Difference from untreated wool | -0.01 | | -0.07 | | -0.09 | |
| | | | | | | |
| 80%/20% Wool/Polyester untreated (n=6,6,5) | 6.03 | 0.12 | -27.76 | 0.51 | 15.43 | 0.19 |
| 80%/20% Wool/Polyester treated (n=18,18,18) | 5.94 | 0.08 | -27.62 | 0.09 | 15.50 | 0.40 |
| Difference from untreated wool | -0.03 | | -0.08 | | -0.23 | |
| | | | | | | |
| 50%/50% Wool/Polyester untreated (n=6,6,5) | 5.93 | 0.06 | -27.86 | 0.32 | 15.55 | 0.22 |
| 50%/50% Wool/Polyester treated (n=18,18,18) | 5.94 | 0.09 | -27.67 | 0.09 | 15.58 | 0.32 |
| Difference from untreated wool | -0.03 | | -0.12 | | -0.15 | |

### Example 9: Nylon extraction from a blend with wool using formic acid

A blend of 50 % wool and 50 % nylon (w/w) was created from ground wool and nylon. The blend was homogenised for 3 min using a Retsch MM 400. Triplicate aliquots (100 mg) of the blend and raw wool were treated with formic acid (98 %, 5 ml) and vortexed for 2 min. The samples were centrifuged for 5 min at 2000 g, the supernatant was decanted off, and a fresh aliquot of formic acid (5 ml) was added to the residue. The samples were vortexed for 2 min before centrifuging was repeated. The supernatant was decanted off and the residue rinsed three times with ethanol (5 ml). Fibres were dried overnight at 60 °C before IRMS analysis. The results are shown in Tables 8 and 9.

**Table 8: Comparison of stable isotope (H and O) measurements for nylon/wool blend samples**

| | δ²H | | δ¹⁸O | |
|---|---|---|---|---|
| | Average | StDev | Average | StDev |
| Wool untreated (n=3,3) | -81.49 | 1.02 | 13.67 | 0.21 |
| Wool treated (n=27,27) | -85.70 | 1.07 | 13.64 | 0.25 |
| Difference | -4.20 | | -0.03 | |
| | | | | |
| Nylon untreated (n=3,2) | -33.72 | 0.25 | 2.65 | 0.24 |
| 50%/50% Wool/Nylon untreated (n=3,3) | - 51.38 | 1.02 | 8.87 | 0.22 |
| 50%/50% Wool/Nylon treated (n=27,27) | -84.30 | 1.08 | 13.44 | 0.20 |
| Difference from untreated wool | -2.80 | | -0.23 | |

**Table 9: Comparison of stable isotope (N, C and S) measurements for nylon/wool blend samples**

| | δ¹⁵N | | δ¹³C | | δ³⁴S | |
|---|---|---|---|---|---|---|
| | Average | StDev | Average | StDev | Average | StDev |
| Wool untreated (n=3,3,3) | 6.04 | 0.08 | -27.61 | 0.06 | 16.07 | 0.36 |
| Wool treated (n=27,27,26) | 5.95 | 0.10 | -27.51 | 0.05 | 16.46 | 0.15 |
| Difference | -0.09 | | 0.09 | | 0.39 | |
| | | | | | | |
| Nylon untreated (n=3,3,2) | -1.46 | 0.03 | -28.34 | 0.06 | 16.54 | 0.12 |
| 50%/50% Wool/Nylon untreated (n=3,3,2) | 2.44 | 0.09 | -28.27 | 0.01 | 16.11 | 0.13 |
| 50%/50% Wool/Nylon treated (n=27,27,27) | 5.95 | 0.10 | -27.67 | 0.15 | 17.02 | 0.14 |
| Difference from untreated wool | -0.08 | | -0.06 | | 0.94 | |

### Example 10: Elastane extraction from a blend with wool using DMF

A blend of 80 % wool and 20 % elastane (w/w) was created from ground wool and elastane. The blend was homogenised for 3 min using a Retsch MM 400). Tripplicate aliquots (100 mg) of the blend and raw wool were treated with DMF (5 ml) and vortexed for 5 min, followed by sonication (5 min at 70 °C) before being placed in a 70 °C water bath for 2 hours. Samples were centrifuged for 5 min at 2000 g, the supernatant was decanted off and 10 % ethanol in DMF solution (5 ml) was added. The samples were vortexed and sonicated prior to heating at 70 °C in a water bath for a further 2 hours. Centrifugating was repeated and the residue washed with 10 % ethanol in DMF solution (5 ml) and vortexed for 5 min. The sample was centrifuged again at 2000 g for 5 min and the residue rinsed twice with ethanol (95 %, 5 ml). Fibres were dried overnight at 60 °C before IRMS analysis. The results are shown in Tables 10 and 11.

**Table 10: Comparison of stable isotope (H and O) measurements for elastane/wool blend samples**

| | δ²H | | δ¹⁸O | |
|---|---|---|---|---|
| | Average | StDev | Average | StDev |
| Wool untreated (n=3,3) | -81.83 | 1.34 | 13.52 | 0.10 |
| Wool treated (n=27,27) | -81.55 | 1.48 | 12.88 | 0.18 |
| Difference | 0.29 | | -0.63 | |
| | | | | |
| Elastane untreated (n=3,3) | -215.79 | 1.09 | 7.81 | 2.09 |
| 80%/20% Wool/Elastane untreated (n=2,2) | -107.35 | 1.31 | 12.91 | 0.10 |
| 80%/20% Wool/Elastane treated (n=27,27) | -81.62 | 1.18 | 13.35 | 0.40 |
| Difference from untreated wool | 0.21 | | -0.17 | |

**Table 11: Comparison of stable isotope (N, C and S) measurements for elastane/wool blend samples**

| | ⁱδ¹⁵N | | δ¹³C | | δ³⁴S | |
|---|---|---|---|---|---|---|
| | Average | StDev | Average | StDev | Average | StDev |
| Wool untreated (n=3,3,3) | 5.53 | 0.10 | -27.62 | 0.04 | 16.67 | 0.17 |
| Wool treated (n=27,27,27) | 5.88 | 0.35 | -27.83 | 0.09 | 15.77 | 2.19 |
| Difference | 0.36 | | -0.21 | | -0.90 | |
| | | | | | | |
| Elastane untreated (n=3,3,3) | -1.79 | 0.19 | -25.88 | 0.12 | 3.36 | 2.35 |
| 80%/20% Wool/Elastane untreated (n=3,3,3) | 5.61 | 0.12 | -27.40 | 0.05 | 14.91 | 0.43 |
| 80%/20% Wool/Elastane treated (n=27,27,23) | 5.46 | 0.42 | -27.87 | 0.08 | 16.97 | 0.78 |
| Difference from untreated wool | -0.07 | | -0.25 | | 0.30 | |

### Example11: Acrylic extraction from a blend with cotton using DMF

A blend of ~50 % w/w was created from ground cotton and acrylic. The blend was homogenised by further milling. Triplicate aliquots (100 mg) of the blend and raw starting materials were treated with DMF (5 ml) and vortexed for 2 min before being placed in a 70 °C water bath for 2 hours. Samples were centrifuged for 5 min at 2000 g. The supernatant was decanted off and 10 % ethanol in DMF (v/v, 5 ml) solution was added. The samples were vortexed prior to heating at 70 °C in a water bath for 2 hours. Samples were centrifuged again for 5 min at 2000 g. The supernatant was decanted off and the residue washed with a fresh DMF/ethanol solution and vortexed for 1 min. The washed sample was centrifuged again at 2000 g for 5 min and the residue rinsed with ethanol (95 %, 5 ml) two more times. Fibres were dried overnight at 60 °C before being prepared for IRMS. The results are shown in Table 12.

**Table 12: Comparison of stable isotope (H, O and C) measurements for acrylic/cotton blend samples**

| **Precision when run in duplicate** | | | | | | |
|---|---|---|---|---|---|---|
| | δ²H | | δ¹⁸O | | δ¹³C | |
| | Average | StDev | Average | StDev | Average | StDev |
| Original (100% cotton) | -37.90 | 0.34 | 25.11 | 0.17 | -26.70 | 0.12 |
| | | | | | | |
| Untreated blend (50% Acrylic, 50% Cotton) | -49.53 | 0.28 | 24.05 | 0.15 | -28.25 | 0.17 |
| Treated blend (50% Acrylic, 50% Cotton) | -38.79 | 0.70 | 25.16 | 0.08 | -26.99 | 0.09 |
| Difference | -0.90 | | 0.05 | | -0.30 | |

### Example 12: Polyester extraction from a blend with cotton using NaOH

A blend of ~50 % w/w was created from ground cotton and polyester. The mixture was further milled to have an approximate particle size of 50-250 µm. Triplicate aliquots (100 mg) were each placed in 15 ml polypropylene centrifuge tubes with NaOH (10% w/v, 5 ml). The centrifuge tubes were vortexed for 5 min and placed in water bath at 90 °C for 4 hours. After centrifuging for 5 min at 2000 g, the supernatant was decanted off and distilled water (5 ml) added to rinse. After vortexing for 5 min then centrifuging for 5 min at 2000 g, the supernatant was decanted off and acetic acid (2% v/v, 5 ml) added to neutralise excess base. After vortexing for 5 min to mix well and then centrifuging for 5 min at 2000 g, the supernatant was decanted off and ethanol (10% v/v, 5 ml) added. After vortexing for 2 min and centrifuging for 5 min at 2000 g the supernatant was decanted off and the residue dried at 60 °C for at least 48 hours before preparing for IRMS. The results are shown in Table 13.

**Table 13: Comparison of stable isotope (H, O and C) measurements for polyester/cotton blend samples**

| | δ²H | | δ¹⁸O | | δ¹³C | |
|---|---|---|---|---|---|---|
| | Average | StDev | Average | StDev | Average | StDev |
| Original (100% cotton) | -41.6 | 0.68 | 25.24 | 0.06 | -26.70 | 0.01 |
| After treatment | -39.83 | 0.66 | 25.20 | 0.12 | -26.92 | 0.15 |
| Difference | -1.79 | | 0.04 | | 0.22 | |
| | | | | | | |
| 50% Polyester, 50% Cotton | -47.30 | 2.24 | 18.85 | 1.54 | -27.95 | 0.31 |
| Treated blend (50% Polyester, 50% Cotton) | -38.06 | 0.95 | 25.37 | 0.10 | -26.70 | 0.06 |
| Difference from raw cotton | 1.76 | | 0.17 | | 0.22 | |

### Example 13: Verification of origin

Data derived from the analysis according to Examples 1 and 2 above of three samples claimed to originate from the USA was compared with data from authentic samples to verify the origin of the samples. The results of the data audit are shown in Figure 1. The trace metal and stable isotope data are presented in multivariate space. The clouds of different shaded balls represent samples from different origins. The large dark ball (upper) is a sample that failed the audit, i.e. is not consistent with the claimed origin while the large lighter balls (lower right) are consistent with the claimed origin i.e. fall within the Australian cluster.

Although the invention has been described by way of example, it should be appreciated that variations and modifications may be made without departing from the scope of the invention as defined in the claims. Furthermore, where known equivalents exist to specific features, such equivalents are incorporated as if specifically referred to in this specification.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by preferred embodiments and optional features, modification and variation of the concepts disclosed herein may be resorted to by those skilled in the art, and that such modifications and variations are considered to be within the scope of this invention as described herein, and as defined by the appended claims.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

## Claims

1. A method for determining the origin of a material comprising a blend of synthetic fibres and natural or semi-synthetic fibres comprising the steps:
a. contacting a sample of the material with an extractant solution to form a solution or suspension comprising i) synthetic fibres and fragments of the synthetic fibres, and ii) a solid residue containing natural or semi-synthetic fibres;
b. separating the solution or suspension formed in step a. from the solid residue;
c. drying the solid residue;
d. determining isotope ratios of one or more of the elements carbon, oxygen, hydrogen, nitrogen, sulfur and strontium in the solid residue, and/or determining the concentrations of one or more trace elements in the solid residue; and
e. comparing the isotope ratios and/or concentrations determined in step d. against data for natural or semi-synthetic fibres of known origin to determine the origin of the material.

2. A method as claimed in claim 1, wherein the natural fibres are fibres of cotton, wool, fur, silk, hemp, linen or jute.

3. A method as claimed in claim 2, wherein the wool originates from sheep, goats or alpaca.

4. A method as claimed in claim 2, wherein the fur originates from rabbits or possums.

5. A method as claimed in any one of claims 1 to 4, wherein the semi-synthetic fibres are fibres of viscose, modal, rayon, acetate, lyocell or cupro.

6. A method as claimed in any one of claims 1 to 5, wherein the synthetic fibres are fibres of polyester, nylon, elastane or acrylic.

7. A method as claimed in any one of claims 1 to 6, wherein the sample of material has beem milled to an average particle size of about 50 to 250 microns before contacting with the extractant solution in step a.

8. A method as claimed in any one of claims 1 to 7, wherein the extractant solution is acetone, ethanol, dimethylformamide (DMF), phenol, dichloromethane (DCM), formic acid, aqueous hydrochloric acid, aqueous sodium hydroxide, or any combination thereof.

9. A method as claimed in any one of claims 1 to 8, wherein step a. comprises one or more cycles of i) agitating the sample in the extractant solution, ii) centrifuging the sample in the extractant solution, and iii) separating the solution from the solid residue.

10. A method as claimed in claim 9, further comprising one or more steps of heating the sample at a temperature in the range 50-90 °C.

11. A method as claimed in any one of claims 1 to 10, wherein the solution formed in step a. is separated from the solid residue by decanting, filtering or aspirating.

12. A method as claimed in claim 1, wherein the synthetic fibre is polyester and the extractant solution is phenol in DCM (20% v/v) or aqueous sodium hydroxide (10% w/v).

13. A method as claimed in claim 1, wherein the synthetic fibre is elastane and the extractant solution is ethanol in DMF (10% v/v).

14. A method as claimed in claim 1, wherein the synthetic fibre is nylon and the extractant solution is aqueous hydrochloric acid (18% w/v) or formic acid (98% v/v).

15. A method as claimed in claim 1, wherein the synthetic fibre is polyester and the extractant solution is aqueous sodium hydroxide (10 % w/v).

## Patentansprüche

1. Verfahren zum Bestimmen der Herkunft eines Materials, das eine Mischung aus synthetischen Fasern und natürlichen oder halbsynthetischen Fasern umfasst, das die folgenden Schritte beinhaltet:
a. Inkontaktbringen einer Probe des Materials mit einer Extraktionslösung, um eine Lösung oder Suspension zu bilden, die i) synthetische Fasern und Fragmente der synthetischen Fasern und ii) einen natürliche oder halbsynthetische Fasern enthaltenden festen Rückstand umfasst;
b. Trennen der in Schritt a. gebildeten Lösung oder Suspension vom festen Rückstand;
c. Trocknen des festen Rückstands;
d. Bestimmen der Isotopenverhältnisse eines oder mehrerer der Elemente Kohlenstoff, Sauerstoff, Wasserstoff, Stickstoff, Schwefel und Strontium im festen Rückstand und/oder Bestimmen der Konzentrationen eines oder mehrerer Spurenelemente im festen Rückstand; und
e. Vergleichen der in Schritt d. bestimmten Isotopenverhältnisse und/oder Konzentrationen mit Daten für natürliche oder halbsynthetische Fasern bekannter Herkunft, um die Herkunft des Materials zu bestimmen.

2. Verfahren nach Anspruch 1, wobei die natürlichen Fasern Baumwoll-, Woll-, Fell-, Seide-, Hanf-, Leinen- oder Jutefasern sind.

3. Verfahren nach Anspruch 2, wobei die Wolle von Schafen, Ziegen oder Alpakas stammt.

4. Verfahren nach Anspruch 2, wobei das Fell von Kaninchen oder Opossums stammt.

5. Verfahren nach einem der Ansprüche 1 bis 4, wobei die halbsynthetischen Fasern Viskose-, Modal-, Rayon-, Acetat-, Lyocell- oder Cuprofasern sind.

6. Verfahren nach einem der Ansprüche 1 bis 5, wobei die synthetischen Fasern Polyester-, Nylon-, Elastan- oder Acrylfasern sind.

7. Verfahren nach einem der Ansprüche 1 bis 6, wobei die Materialprobe vor dem Inkontaktbringen mit der Extraktionslösung in Schritt a auf eine durchschnittliche Partikelgröße von etwa 50 bis 250 Mikrometer gemahlen wurde.

8. Verfahren nach einem der Ansprüche 1 bis 7, wobei die Extraktionslösung Aceton, Ethanol, Dimethylformamid (DMF), Phenol, Dichlormethan (DCM), Ameisensäure, wässrige Salzsäure, wässriges Natriumhydroxid oder eine beliebige Kombination davon ist.

9. Verfahren nach einem der Ansprüche 1 bis 8, wobei Schritt a einen oder mehrere Zyklen von i) Rühren der Probe in der Extraktionslösung, ii) Zentrifugieren der Probe in der Extraktionslösung und iii) Trennen der Lösung von dem festen Rückstand beinhaltet.

10. Verfahren nach Anspruch 9, das ferner einen oder mehrere Schritte des Erhitzens der Probe auf eine Temperatur im Bereich von 50 bis 90 °C beinhaltet.

11. Verfahren nach einem der Ansprüche 1 bis 10, wobei die in Schritt a gebildete Lösung durch Dekantieren, Filtrieren oder Absaugen vom festen Rückstand getrennt wird.

12. Verfahren nach Anspruch 1, wobei die synthetische Faser Polyester ist und die Extraktionslösung Phenol in DCM (20 % v/v) oder wässriges Natriumhydroxid (10 % w/v) ist.

13. Verfahren nach Anspruch 1, wobei die synthetische Faser Elastan ist und die Extraktionslösung Ethanol in DMF (10 % v/v) ist.

14. Verfahren nach Anspruch 1, wobei die synthetische Faser Nylon ist und die Extraktionslösung wässrige Salzsäure (18 % w/v) oder Ameisensäure (98 % v/v) ist.

15. Verfahren nach Anspruch 1, wobei die synthetische Faser Polyester ist und die Extraktionslösung wässriges Natriumhydroxid (10 % w/v) ist.

## Revendications

1. Un procédé de détermination de l'origine d'un matériau composé d'un mélange de fibres synthétiques et de fibres naturelles ou semi-synthétiques, comportant les étapes consistant à :
a. mettre en contact un échantillon du matériau avec une solution d'extraction pour former une solution ou une suspension comprenant i) des fibres synthétiques et des fragments de ces fibres synthétiques, et ii) un résidu solide contenant des fibres naturelles ou semi-synthétiques ;
b. séparer la solution ou suspension formée en étape a. du résidu solide ;
c. sécher le résidu solide ;
d. déterminer les rapports isotopiques de l'un ou de plusieurs des éléments suivants : carbone, oxygène, hydrogène, azote, soufre et strontium dans le résidu solide, et /ou déterminer les concentrations d'un ou de plusieurs éléments traces dans le résidu solide ; et
e. comparer les rapports isotopiques et /ou les concentrations déterminées à l'étape d. avec des données sur des fibres naturelles ou semi-synthétiques d'origine connue pour déterminer l'origine du matériau.

2. Un procédé selon la revendication 1, dans lequel les fibres naturelles sont des fibres de coton, laine, fourrure, soie, chanvre, lin ou jute.

3. Un procédé selon la revendication 2, dans lequel la laine provient de moutons, chèvres ou alpagas.

4. Un procédé selon la revendication 2, dans lequel la fourrure provient de lapins ou d'opossums.

5. Un procédé selon une quelconque des revendications 1 à 4, dans lequel les fibres semi-synthétiques sont des fibres de viscose, modal, rayonne, acétate, lyocell ou cupro.

6. Un procédé selon une quelconque des revendications 1 à 5, dans lequel les fibres synthétiques sont des fibres de polyester, nylon, élasthanne ou acrylique.

7. Un procédé selon une quelconque des revendications 1 à 6, dans lequel l'échantillon de matériau a été broyé à une taille de particule moyenne d'environ 50 à 250 microns avant d'être mis en contact avec la solution d'extraction en étape a.

8. Un procédé selon une quelconque des revendications 1 à 7, dans lequel la solution d'extraction est de l'acétone, de l'éthanol, du diméthylformamide (DMF), du phénol, du dichlorométhane (DCM), de l'acide formique, de l'acide chlorhydrique aqueux, de l'hydroxyde de sodium aqueux ou toute combinaison de ceux-ci.

9. Un procédé selon une quelconque des revendications 1 à 8, dans lequel l'étape a. comprend un ou plusieurs cycles de i) agitation de l'échantillon dans la solution d'extraction, ii) centrifugation de l'échantillon dans la solution d'extraction et iii) séparation de la solution du résidu solide.

10. Un procédé selon la revendication 9, comprenant en sus une ou plusieurs étapes de chauffage de l'échantillon à une température comprise entre 50 et 90 C.

11. Un procédé selon une quelconque des revendications 1 à 10, dans lequel la solution formée en étape a. est séparée du résidu solide par décantation, filtration ou aspiration.

12. Un procédé selon la revendication 1, dans lequel la fibre synthétique est du polyester et la solution d'extraction est du phénol dans du DCM (20% v/v) ou de l'hydroxyde de sodium aqueux (10% p/v).

13. Un procédé selon la revendication 1, dans lequel la fibre synthétique est de l'élasthanne et la solution d'extraction est de l'éthanol dans du DMF (10% v/v).

14. Un procédé selon la revendication 1, dans lequel la fibre synthétique est du nylon et la solution d'extraction est de l'acide chlorhydrique aqueux (18% p/v) ou de l'acide formique (98% v/v).

15. Un procédé selon la revendication 1, dans lequel la fibre synthétique est du polyester et la solution d'extraction est de l'hydroxyde de sodium aqueux (10 % p/v).
